# EUROPEAN PATENT APPLICATION

(11) **EP 1 479 344 A1**
(43) Date of publication of application: **24.11.2004**
(21) Application number: 03011576.0
(22) Date of filing: 22.05.2003
(51) Int. Cl.: A61B 10/00, A61B 5/00

(54) **Direct monitoring of interstitial fluid composition**

(71) Applicant: Roche Diagnostics GmbH, 68305 Mannheim (DE); F.HOFFMANN-LA ROCHE AG, 4070 Basel (CH)
(72) Inventor: Haar, Hans-Peter, 69168 Wiesloch (DE); Meacham, George Bevan Kirby, Shaker Heights, Ohio 44122 (US); List, Hans, 64754 Hesseneck-Kailbach (DE)
(74) Representative: Jung, Michael, Dr.

(57) **Abstract**

The present invention concerns a system for sampling body fluids, comprising an implantable catheter for introduction into tissue, a pump means for transporting a volume of said body fluid out of the catheter, a volume determination unit for determining said volume of fluid and a control means (70) for controlling the pump means based on the determined volume of body fluid. Alternatively to volume determination and control the invention proposes to sample with an overall flow rate of below 10 nanoliters per minute or to sample below 50 nanoliters per sampling event.

## Description

### FIELD OF THE INVENTION

The invention concerns devices for sensing the concentration of chemical constituents in body fluid such as interstitial fluid, including but not limited to glucose. The devices also relate to systems for continuously monitoring and reporting body fluid constituents in response to the sensed concentrations to permit aggressive therapy, and provide the clinical benefits of such therapy.

### BACKGROUND OF THE INVENTION

Metabolic processes of living organisms depend on maintaining the concentration of chemical compounds, including glucose, within certain limits in the interstitial fluid surrounding living cells. This fluid occupies perhaps 20% of the tissue volume, and the balance of the volume is taken up by cells. The fluid actively flows through the tissue. The flow source is plasma filtered through the arterial capillary walls and leaked through the venous capillaries, and the sink is flow into the venous capillaries and the lymphatic system. The flow rate is reported to be 0.36*10⁻² ml/sec*ml of tissue, and results in a complete change of fluid in each milliliter of tissue in less than 5 minutes. The cells absorb required materials, including oxygen and glucose, from this flowing fluid. At the same time, waste products including carbon dioxide, are released into the fluid. This flow provides one mechanism for bringing oxygen and glucose to the cells. Diffusion of oxygen and glucose, both small molecules, provides a second transfer mechanism. As a result of these transfer mechanisms, the concentration of glucose in the interstitial fluid is very nearly the same as in the arterial capillaries.

Although the glucose concentration of interstitial fluid is similar to that of blood, it has important differences from blood. It does not contain blood cells, since these do not leave the capillaries, and it does not clot. The static pressure is at or below atmospheric, while the capillary blood pressure is on the order of 30 mmHg above atmospheric. The interstitial fluid protein content is lower than that of the blood plasma, and this creates an inward osmotic pressure across the capillary walls. This osmotic pressure is an important component of the overall pressure and flow balance between the capillaries and the tissue.

Interstitial fluid is an attractive target for glucose measurement. A suitable measurement system is e.g. described in US 5,582,184. Since it floods the tissue, it maybe accessed by a subcutaneous probe that does not disrupt capillaries. Blood samples, in contrast, are typically obtained by cutting through the skin, subcutaneous tissue and capillaries with a lancet and collecting the blood that is expelled before clotting mechanisms stop the flow and initiate the healing process. The small size of capillaries (10 to 20 microns diameter) precludes insertion of a catheter for less traumatic access.

A number of well-known methods are available to measure glucose in liquid samples. Methods using dry coated test strips that change color as a function of fluid glucose concentration when a sample is applied are particularly advantageous. This color change may be automatically measured by an optical module, and used to determine and report the glucose concentration. This value is an absolute measurement, and may be used to guide insulin therapy. No calibration solutions are required, and sample volumes as low as 5 to 10 nanoliters have been shown to provide reliable measurements. Such test elements are e.g. described in US 6,036,919. Alternatively to measurements with dry coated test strips, other measurement techniques such as electrochemical flow-cells or infrared analysis can be employed to determine analyte concentration in sampled body fluid.

Ordinarily, living organisms employ homeostatic mechanisms to control the concentration of glucose and other constituents in the blood and interstitial fluid, since concentrations outside these limits may cause pathology or death. In the case of glucose, specialized pancreatic cells sense blood glucose levels, and release insulin as glucose increases. Insulin receptors in other tissues are activated, and increase glucose metabolism to reduce the glucose level. Type I diabetes is caused by death of insulin producing cells, and Type II diabetes is caused by reduced insulin receptor sensitivity. In both cases, the result is excess blood glucose. The blood glucose may be controlled, particularly in the case of type I diabetes, by administration of insulin. While this is effective in reducing glucose, the dose must be quantitatively matched to the amount of glucose reduction required. An insulin overdose may lead to very low blood glucose, and result in coma or death. Clinical trials have conclusively proven that stabilizing glucose levels through frequent measurement of blood glucose and appropriate insulin administration reduces the pathology of diabetes.

Such strict glucose control is, however, a difficult regimen. The established method of glucose measurement uses small samples of blood from arterial capillaries obtained by pricking the finger and expressing the sample onto a disposable test strip. A meter device is used to read the test strip, and report a quantitative blood glucose concentration. The appropriate dose of insulin is then calculated, measured out and administered with a hypodermic needle. The overall process is both painful and technically demanding, and cannot be sustained by many patients.

Automated insulin delivery devices help some patients maintain the regimen. These are small, wearable devices that contain a reservoir of insulin, an insulin pump, a programmable control and a power source. Insulin is delivered to the subcutaneous tissue on a programmed dosage schedule through a catheter implanted in the subcutaneous tissue. The schedule is set to provide the approximate baseload requirements of the particular patient. The patient then makes periodic blood glucose measurements and adjusts the dosage to correct his glucose level. The catheter remains in the subcutaneous tissue for a day or two, after which it is replaced by a new catheter in a different location. This periodic catheter change is needed to prevent tissue reactions that encapsulate the catheter, and to minimize the chance of infection where the catheter passes through the skin. While this reduces or eliminates hypodermic injections, frequent finger pricking and test strip measurements are still required.

It is recognized that a device to measure glucose continuously without requiring finger stick blood samples would be a major step forward. At minimum, it would provide the patient with timely information to make insulin injections or adjust his insulin pump. Further, it could be used to control an insulin pump automatically to mimic the body's natural homeostatic glucose regulation system. Successful development of such measuring systems has proven elusive, however. Noninvasive optical or chemical devices provide relative measurements at best, and have not proven capable of providing an absolute measurement that is reliable enough to determine the insulin dose. Transcutaneous or totally implanted glucose probes based on electrochemical or spectroscopic principles provide absolute measurements of the interstitial fluid glucose. This measurement tracks blood glucose, and may be used as a basis for insulin administration. The problem is probe encapsulation as well as aging of the employed sensors that degrades the measurement in a matter of a few days.

There is, therefore, a need for a simple and robust means and apparatus for measuring the absolute concentration of chemical constituents, particularly glucose but not limited to glucose, in the subcutaneous interstitial fluid. Continuous measurement of analyte concentrations in body fluids is e.g. described in WO 02/062210 and WO 00/22977. While the first document describes measurement based on sampling minute amounts of body fluid by an implanted catheter and applying the obtained fluid to dry analytical test elements, the latter document describes a similar system which employs electrochemical cells for measurement. According to the present invention it has been found that such systems are able to obtain fluid over some hours but that it may be hard to obtain fluid for measurement over several days. WO 00/22977 describes an electroosmotic enhancement of fluid sampling. This however, may not be a reliable method and it complicates the sampling device. According to the present invention it was found that the amount of fluid which can be sampled from a sampling site into which a catheter is implanted is limited. Even strong suction or other means are unsuccessful to sample further fluid after this maximum amount of obtainable fluid has been reached. The sampling and measurement technique described in WO 02/062210 allows the analysis to be conducted with a few nanolitres only. The problem of a limited amount of body fluid as obtainable from a single sampling side is solved according to the present invention by detecting the volume which is sampled and to control the sampling process in a way that only the fluid amount necessary for a single determination is sampled each time. The maximum obtainable body fluid volume from a single sampling side is therefore devided up into a larger number of sampling events and monitoring of body fluid concentrations is therefore available over several days. Alternatively to monitoring the sampled amount it is also possible to decrease the volume which is sampled in a single sampling event to below 50 nl, prefereably to below than 15 nl. This too enables monitoring of analyte concentration over some days by successive samplings.

### SUMMARY OF THE INVENTION

An aspect of the invention is provision of a system to draw fluid samples of controlled volume from the catheter. Preferably the volumes are sampled at specified time intervals and deposed on a test element. Such a test element is e.g. a conventional colorimetric test strip. Test strips suitable for use in the present invention are e.g. described in US 6,039,919. The strips have a test area which is impregnated with a reagent system so that the color of the area is changed based on reaction with the analyte to be determined. A further preferred type of test elements employs reagents which upon reaction with analyte from the sample allow fluorescence measurement. Such test chemistries and test elements basing thereon are e.g. describend in german patent application 10221845.5 . In general within the present invention test elements are preferred which can be read by optical methods. With optical detection methods the zone where sample has been applied can be located and analysis can be based on changes of this region. When a testing area is employed which is larger than a zone wetted by sample fluid then positioning of test area relative to the fluid outlet and relative to the detection unit is rather uncritical.

In acordance with the present invention test elements are prefereably employed which are irreversible or disposable in the sense that a zone to which a sample fluid is applied reacts and gives rise to a detectable change which relates to an analyte concentration. It is a one-way mesasurement, the very same test zone is never used again. The test elements, however, may have multiple testing areas or testing areas large enough to provide for multiple test zones. The use of irreversible/disposable test areas has the advantage that a signal drift can be avoided or at least detected. In contrast thereto non-disposible sensors, as e.g. flow cell sensors, have a signal drift that is hard to account for. Disposable dry chemistry test elements are very reliable and accurate.

In the present invention prefereably test elements are employed which provide multiple test zones so that numerous testings can be made by using the same test element. The term test area means an area on a test element where reagent is present so that testing can be made. Contrary the term test zone means the zone within which testing is done after sample application.

Test areas advantageously can be provided on an extended carrier as e.g. a tape contrary to providing each test area on an individual carrier. Test elements for multiple testing may have testing areas which are separated one from the other. Alternatively test elements can be employed which have testing areas in which more than one testing can be made. It has to be understood that the location for sample application does not need to be a predefined area. When using testing areas that are larger than an area wetted by a single sampling event the actual zone for testing is selected by sample application.

Especially tapes having one or more test areas allow convenient transport of fresh test zones into a contact zone where liquid sample is then applied to the test zone. For a more detailed description of such tape based systems reference is made to WO 02/062210. Further test elements in the form of discs are preferred where transport of a fresh testing area into a sample receiving location can be made by rotating the disc.

Catheters suitable for the present invention are primarily types with a single channel which do not employ external liquids as perfusion fluid to sample body fluid. The sampling catheter may be a steel needle with bores at its distal portion that is implanted in interstitial tissue. Suitable catheters are e.g. described in WO 02/062210 and WO 00/22977. Further a system is proposed which employs a double channel catheter. While from one channel a bolus of body fluid is sampled the second channel which communicates with the first one, is filled with air or gas. In the time between successive fluid withdrawals from the catheter the second channel is filled with body fluid which enters through apertures or holes.
Alternative embodiments are used to control the volume sampled from the catheter. In a first embodiment pressure level and application time are controlled to deliver the specified volume through a fixed flow restriction. The optical measuring system may be used to determine the actual sample volume delivered as a feedback signal to adjust the pressure level and time interval. Such an optical measuring system can be provided by a CCD chip onto which an image of the test zone is projected. Evaluation of the image shows the area wetted by sample liquid and from this area the amount of sample fluid as received on the test zone can be determined. In a second embodiment a moving shuttle limits the volume of the sample withdrawn. In a third embodiment a positive displacement plunger pump withdraws and delivers the specified sample volume. In a fourth embodiment an elastomeric conduit between the catheter and the test strip cooperates with mechanical actuators to withdraw and deliver the specified sample volume by a peristaltic pumping process. In a fifth embodiment a dual channel catheter provides an equilibrated sample upon demand with a shorter time delay than is possible with the single channel catheters in the previous four embodiments.

According to the invention the physiological effects of the sample withdrawal process are minimized. The size of the samples (5 to 10 nanoliters for example), and the sampling time intervals (5 minutes for example) result in an interstitial fluid withdrawal rate that is small compared to the flow rate through the tissue surrounding the catheter. This minimizes the effect of the fluid withdrawal on the flow rate and chemical composition of the interstitial fluid. It has been found that overall flow rates in the order of or below below 10 nanoliter per minute are well suited to minimize the effects on fluid composition and provide a long time over which concentration monitoring can be made at the same implantation site.

According to a further aspect of the invention pump means are disclosed which are advantageous for withdrawing minute amounts of fluid from an implanted catheter.

### BRIEF DESCRIPTION OF THE DRAWINGS

The subject of the invention will now be described in terms of its preferred embodiments with reference to the accompanying drawings. These embodiments are set forth to aid the understanding of the invention, but are not to be construed as limiting.

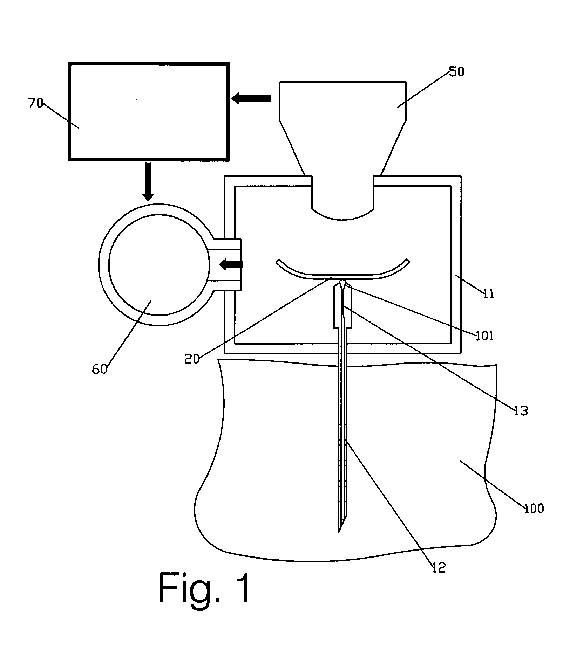

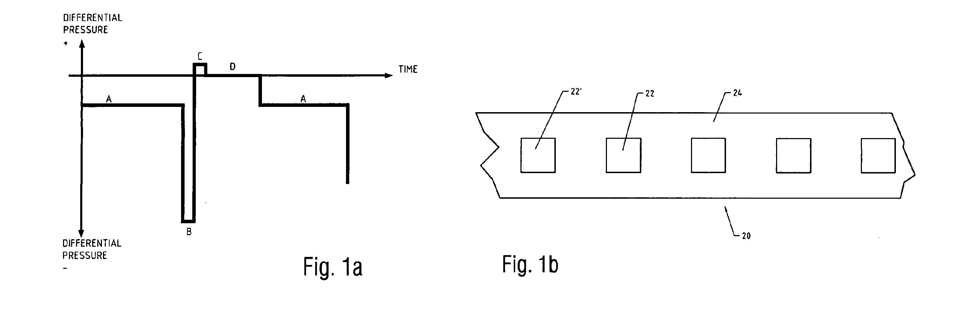

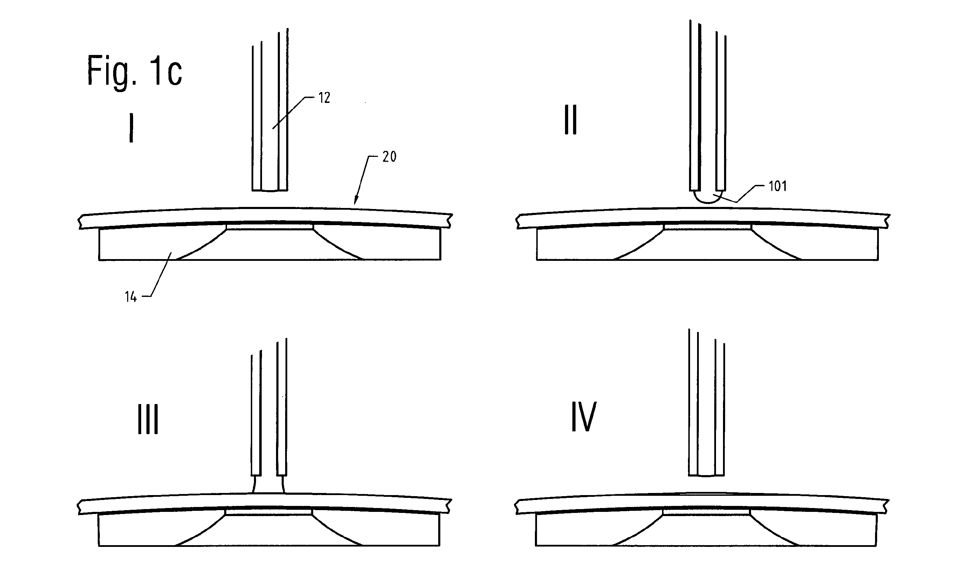

Fig. 1 shows a first embodiment of a system for direct monitoring of interstitial fluid composition.
Fig. 1 a depicts a differential pressure over time diagram
Fig. 1 b shows a tape based test element
Fig. 1 c depicts phases of fluid application to a test element
Figures 2, 3 and 4 show a series of views illustrating the operation of the first embodiment shown by Fig. 1.
Fig. 5 shows a second embodiment of a system for direct monitoring of interstitial fluid composition.
Figures 6, 7 and 8 show a series of views illustrating the operation of the second embodiment shown by Fig. 5.
Fig. 9 shows a third embodiment of a system for direct monitoring of interstitial fluid composition.
Figures 10, 11 and 12 show a series of views illustrating the operation of the third embodiment shown by Fig. 9
Fig. 13 shows a fourth embodiment of a system for direct monitoring of interstitial fluid composition.
Figures 14, 15, 16 and 17 show a series of views illustrating the operation of the fourth embodiment shown by Fig. 13.
Fig. 18 shows a fifth embodiment of a system for direct monitoring of interstitial fluid composition.
Figures 19, 20 and 21 show a series of views illustrating the operation of the fifth embodiment shown by Fig. 18.

### DETAILED DESCRIPTION OF PREFERRED EMBODIMENTS

### EMBODIMENT 1

Fig. 1 shows a fluid handling system that uses vacuum within the device housing (11) to draw sample liquid from the catheter (12) and deposit it on a test area (20). The catheter provides a fluid passage between the subcutaneous tissue (100) and a test strip. It may include a flow restriction (13) to make the flow less dependent on the flow resistance of the tissue. A segment of the test tape concerning the test area is shown. It is part of a roll that is advanced so that a fresh test area is brought to the catheter outlet for each measurement. In addition to providing the colormetric measurement, the test area absorbs the sample. The used test element, including the sample fluid, is stored and discarded after one to two days as part of a disposable module (e.g. a magazine).

An optical reading module (50) containing a light source and a photosensor is positioned to measure the color change of the test zone when it is wetted with the sample. It may also includes an imaging function to confirm that liquid has been applied to the test element, and to provide feedback to control the sample quantity. As shown in figure 1 sample (101) is applied from an outlet of the catheter to the front side of the test area and optical evaluation of the deposed sample volume as well as color determination for analyte measurement is made from the backside of the test element.

The vacuum pump (60) reduces the pressure inside the housing (11) that contains the test element to draw fluid out of the catheter. A control module (70) regulates the pump operating time and / or pressure level so that fluid is withdrawn out of the catheter. A withdrawn fluid droplet (101) is contacted with the test element. The control module may use data from the optical reading module as a feedback signal.

Figure 1 a shows a pressure over time course which can be employed in a system according to the present invention, especially in the system of figure 1. In a first time period (A) the pressure in the catheter is lowered by approx. 10 to 50 mbar relative to its surrounding. During this phase which normally is in the range of some minutes fluid is drawn form the tissue into the catheter but pressure is set so, that no fluid leaves the catheter outlet. In time period (B) the differential pressure is increased to a range of approx. 50 to 300 mbar so that fluid withing the catheter is withdrawn from the catheter outlet. After phase (B) the differential pressure now can be set to a positive pressure for a short time so that liquid flow out of the catheter is actively stopped. When no sample is needed in the next time the differential pressure can be set to near zero so that no tissue fluid is drawn from the tissue into the catheter. When, however, a further measurement is desired the differential pessure in the catheter is again lowered as in period (A) beforehand so that the catheter is filled with fresh tissue fluid representing the actual analyte concentration within the tissue fluid.

Figure 1 b shows a test element in form of a tape that can be employed in the embodiment of figure 1 as well as in other embodiments of the present invention. The tape has a carrier layer from e.g. polyethylene onto which a reagent is applied. A reagent layer is applied to the carrier layer without intersecting portions in longitudinal direction. Alternatively separate reagent layers can be applied to the carrier tape by e.g. a double sided adhesive. Bonding of the reagent layer to the carrier tape can be made by application of the reagent matrix directly to the carrier, e.g. by screen printing. Alternative techniques as bonding with adhesives, double sided adhesive tapes,are well known in the art. The reagent layer provides a testing area (21) onto which sample liquid can be applied. Figure 1 b depicts individual test zones (22) within the testing area. Prefereably a mesh layer is employed which is located above the reagent layer. Such mesh layers are e.g. described in EP 0 995 992. As can be seen from figure 1 b the test zones are wetted by sample liquid in a way that rectangular or nearly quadratic test zones are formed. This is particularly useful for optical examination by e.g. CCD arrays. Therefore mesh layers with quadratic or rectangular meshes are preferred. Preferred mesh sizes are in the range of 10 to 50 micrometer. Further, as described in EP 0 995 992 the mesh layer or its upper portion can be impregnated with wetting agents to improve fluid takeup by the test element. Particularly preferred wetting agents are N-oleoyl-sarcosinates.

Figure 1 c shows the phases of fluid application to the test element. Drawing I depicts the outlet of the catheter above the test element in a phase where no liquid transfer occurs (e.g. phase (A) or (D) of figure 1 a). The depicted test element portion (a test tape in the actual case) rests on a carrier plate (14). The depicted plate has an aperture to allow optical inspection of the test zone from below. This requires either recesses in the carrier tape or a transparent carrier tape at locations where measurement is desired. However, optical inspection alternatively can be made from the upper side (side of sample application) as well. The carrier plate prefereably can be moved relative to the catheter outlet in a way to increase or to decrease the distance between outlet and test zone. By such a movement contact between liquid at the catheter outlet and the upper side of the test element can be achieved. However, figure 1 c shows that such a relative movement can be avoided if desired. As depicted in drawing II fluid emerges from the catheter outlet and contacts the test element when the formed drop at the outlet is large enough (see drawing III). Due to the suction activity of the test element the fluid is withdrawn from the catheter outlet so that the liquid bridge between test element and catheter outlet breaks. A situation according to drawing IV is now achieved.

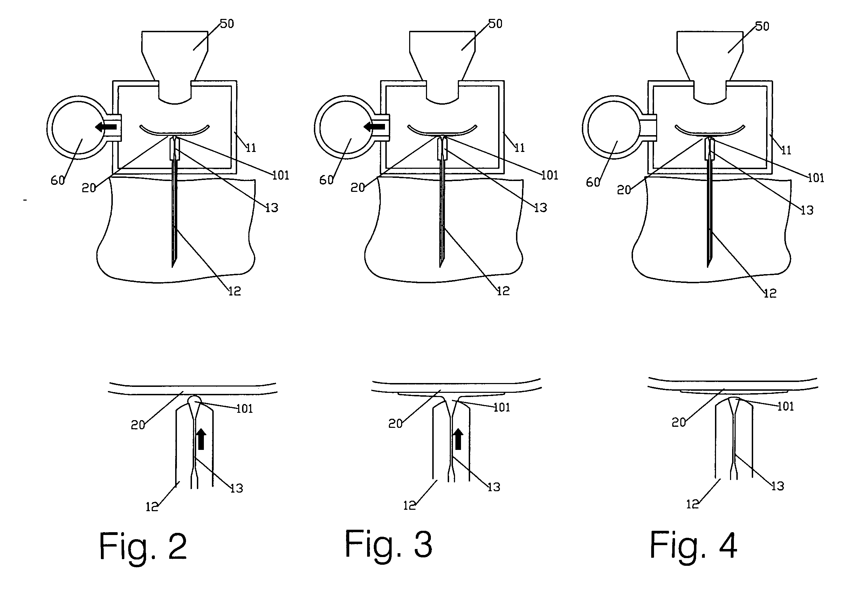

Operation of embodiment 1 is further illustrated in Figures 2, 3 and 4. In Fig. 2 a fresh test zone is moved adjacent to the end of the sample catheter, and the vacuum pump is turned on. The reduced pressure in the housing draws liquid from the subcutaneous tissue and forms a droplet that contacts the test zone. The optional flow restriction limits the flow rate at a given pressure. In Fig. 3 fluid is drawn onto the test zone by capillary action, and forms a wet spot within the field of view of the optical measuring module. This wet spot in the testing area defines the testing zone where actual reaction of analyte with the dry analytical reagent occurs. In Fig. 4 the vacuum pump is then turned off, and the droplet connecting the catheter to the test strip breaks.

In Fig. 4 the wet spot changes color as a function of fluid glucose concentration. This color change is measured by the optical module, and is used to calculate the glucose concentration. This value is reported, and may be used to guide insulin therapy.

Several variations of embodiment 1 system operation are possible. In the first variation a given level of vacuum may be applied for a given time period. If the flow restriction is the primary resistance to flow, and interstitial fluid properties are relatively constant, the sample size will be repeatable. In a second variation the optical module may measure the wet spot size as it forms, and provide feedback to the control module to adjust the vacuum pump. In the third variation the optical module may measure the final wet spot size, and provide feedback to the control module to adjust the vacuum pump for the next sample. As described in WO 02/062210 the spot size on the test zone can be correlated to the sample volume.

### EMBODIMENT 2

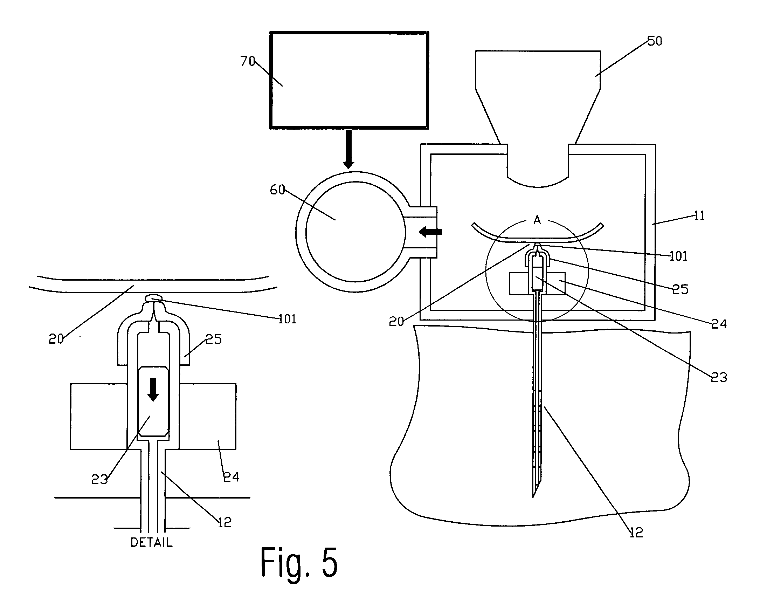

Fig. 5 shows a second embodiment of a fluid handling system that uses vacuum within the device housing to draw sample liquid from the catheter and deposit it on the test strip. A shuttle (23) in the catheter conduit meters out a specified sample volume each time vacuum is applied. The catheter (12) provides a fluid passage between the subcutaneous tissue and the test element(20). It includes an enlarged bore section that contains a small ferromagnetic shuttle (23). The shuttle has a small but non-zero radial clearance with the bore. An external permanent magnet (24) acts on the shuttle and biases it away from the catheter outlet. A spring in the fluid could also be used, but would result in more dead volume. A rubber duckbill check valve (25) is attached to the end of the catheter to allow fluid flow out, but prevent airflow in. The vacuum pump reduces the pressure inside the housing that contains the test element to draw fluid out of the catheter. The control module (70) regulates the vacuum pump (60), but does not use optical information as input.

The test element and optical module (50) are the same as in the description of first embodiment 1.

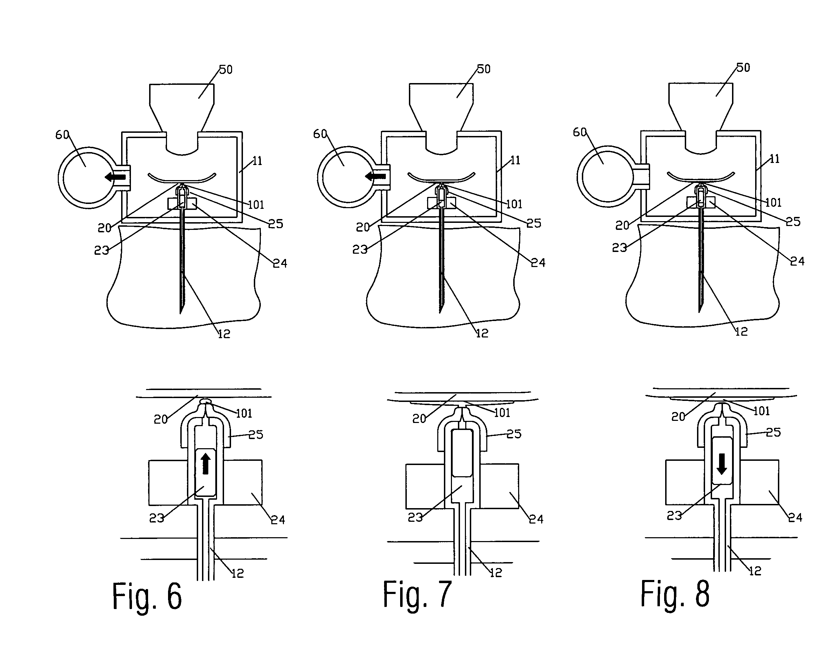

Operation is illustrated in Figures 6, 7 and 8. In Fig. 6 a fresh test area is moved adjacent to the end of the sample catheter (12), and the vacuum pump is turned on. The reduced pressure in the housing (11) draws liquid from the subcutaneous tissue. The liquid flows through the duckbill check valve, and forms a droplet that contacts the test zone. In Fig. 7 the fluid flow moves the shuttle against the magnetic force, since the leakage flow around the shuttle is small relative to the total flow. When the shuttle reaches the end of its travel (FIG. 8), it blocks the flow as long as the vacuum remains. The result is that a defined fluid volume is applied to the test area without active feedback or control. The fluid application time is a few seconds. After the test, the vacuum pump is turned off. The permanent magnet can then pull the shuttle back to the original position shown in Fig. 6. The duckbill check valve prevents airflow in, and fluid flows around the shuttle. The shuttle return time may be a few minutes because of the small radial clearance.

Glucose measurement proceeds as in the first embodiment in Fig. 1.

### EMBODIMENT 3

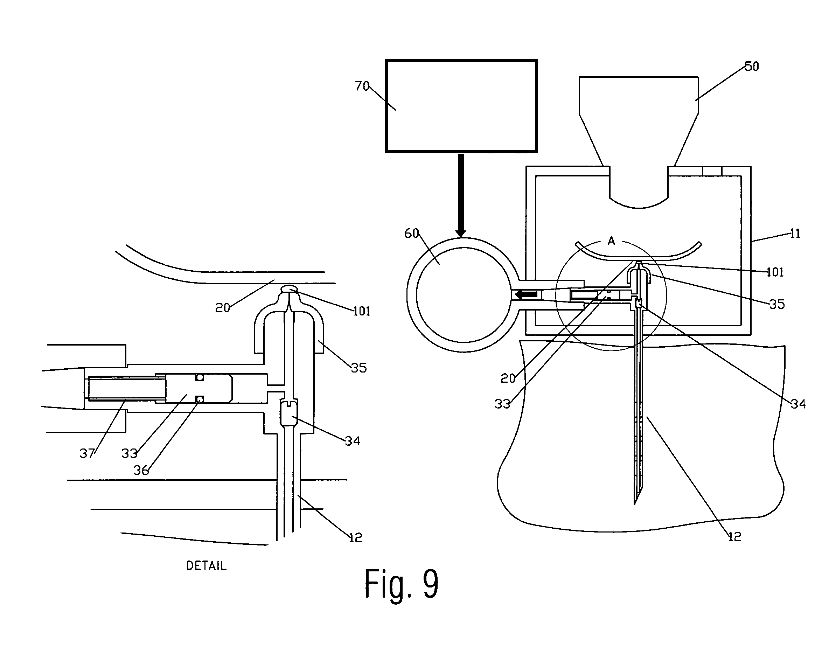

Fig. 9 shows a third embodiment of a fluid handling system that uses vacuum to operate a plunger pump to draw a specified sample volume from the catheter (12) and deposit it on a test area. Unlike the previous embodiments, there is not a vacuum within the device housing (11). The catheter provides a fluid passage between the subcutaneous tissue and the test element. A side passage connects to a pump plunger (33), and a check valve (34) prevents backflow from the plunger to the tissue. A rubber duckbill check valve (35) is attached to the end of the catheter to allow fluid flow out, but prevents airflow in. The pump plunger displaces a single sample volume, and is spring-biased to expel fluid. The plunger includes a sliding seal (36). It should be noted that the spring (37) is not within the fluid, and therefore does not add to the dead volume. The vacuum pump (60) reduces the pressure behind the plunger and draws it back against the spring. The housing itself is vented to atmosphere. The control module (70) regulates the vacuum pump to stroke the pump plunger.

The test element and optical module (50) are the same as in the first embodiment in Fig. 1.

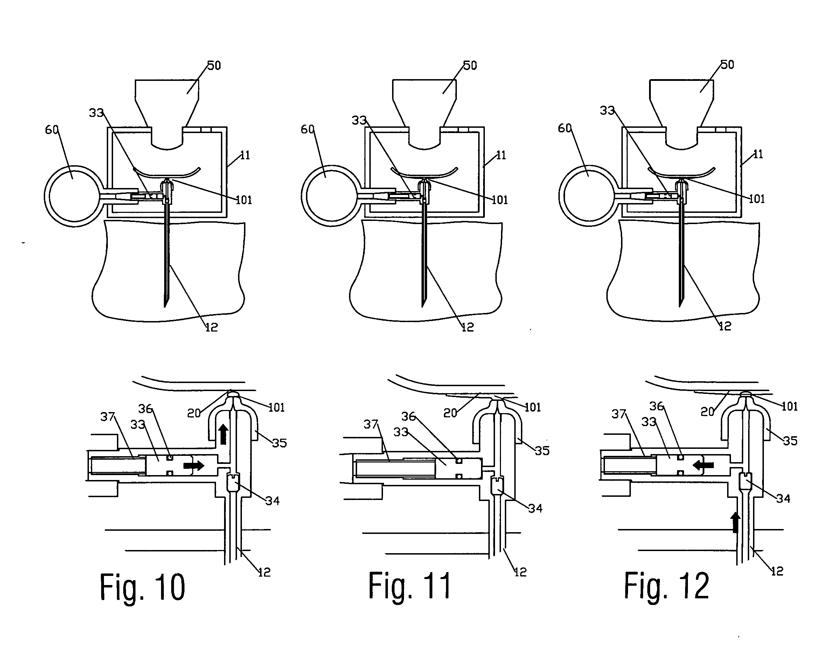

Operation is illustrated in Figures 10, 11 and 12. In Fig. 10 a fresh test area is moved adjacent to the end of the sample catheter, and the vacuum pump (60) is turned off. This releases the pump plunger so that fluid is displaced into the catheter. In Fig. 11 the liquid flows through the duckbill check valve, and forms a droplet that contacts the test zone. The check valve prevents flow back into the tissue. In Fig. 12 the piston plunger reaches the end of its travel and the flow stops. The result is that a defined fluid volume is applied to the test zone without active feedback or control. The fluid application time is a few seconds.

After the test, the vacuum pump is turned on to pull the piston plunger back to the original position shown in Fig. 9. The duckbill check valve prevents airflow in, and fluid flows from the interstitial tissue to fill the pump chamber through the open check valve. The piston plunger return time may range from seconds to a few minutes depending on how quickly the tissue releases fluid.

Glucose measurement proceeds as in the first embodiment in Fig. 1. While vacuum actuation of the pump plunger is shown, a variety of mechanical or fluidic means may be used.

### EMBODIMENT 4

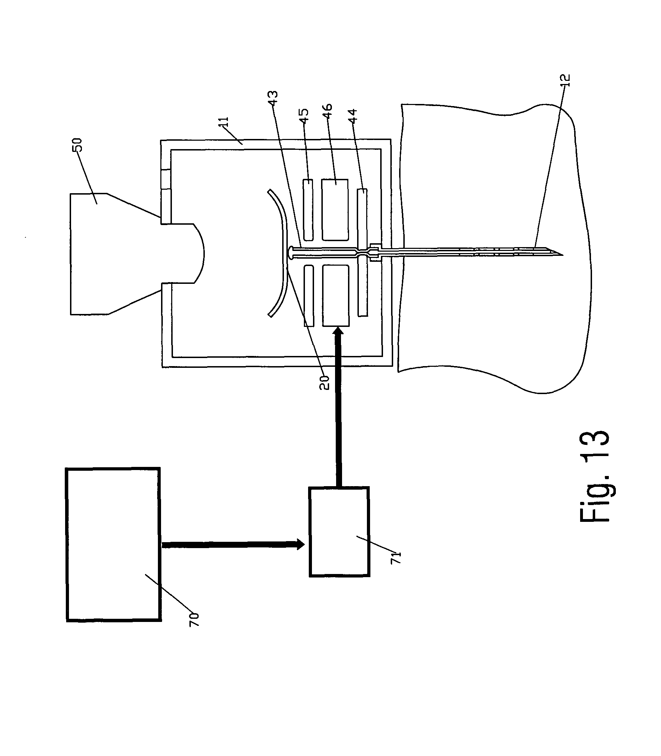

Fig. 13 shows a fourth embodiment of a fluid handling system that uses a peristaltic pump to draw a specified sample volume from the catheter (12) and deposit it on the test element. The catheter provides a fluid passage between the subcutaneous tissue and the peristaltic pump rubber pinch tube (43). The pinch tube continues the fluid passage from the catheter to the test zone. The rubber pinch tube passes between three pairs of clamps that may be individually actuated to squeeze the tube flat. Two pairs function as pinch valves and one pair functions as a displacement pump. The control module (70) sequences a mechanical actuator (71) that moves the clamps.

The test zone and optical module systems are the same as in the first embodiment in Fig. 1.

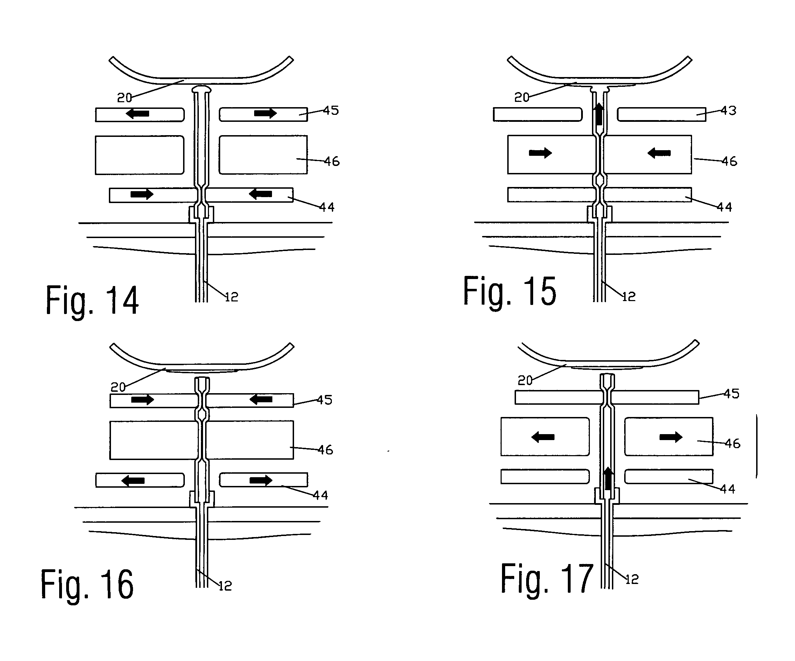

Operation is illustrated in Figures 14, 15, 16 and 17. In Fig. 14 a fresh test area is moved adjacent to the end of the rubber pinch tube, and the first pinch valve (44) is closed and the second pinch valve (45) is opened. In Fig. 15 the pump clamps (46) then squeeze the center section of the rubber pinch tube flat, pumping fluid to the test zone. The first pinch valve remains closed to prevent flow back into the tissue. The result is that a defined fluid volume is applied to the test zone without the need for an active feedback or control. However, sampled fluid volume may be determined and the pump clamps may be controlled with regard to the degree of closing that a desired amount of fluid is expelled. The fluid application time is a few seconds. After the test (Fig. 16), the second pinch valve is closed and the first pinch valve is opened. In Fig. 17 the pump clamps open, and the rubber pinch tube begins to expand to its original round shape. This expansion creates a vacuum that draws additional sample fluid from the interstitial tissue through the catheter to refill the tube. The second pinch valve prevents airflow in. The refilling time may range from seconds to a few minutes depending on how quickly the tissue releases fluid.

Glucose measurement proceeds as in the first embodiment in Fig. 1.

### Embodiment 5

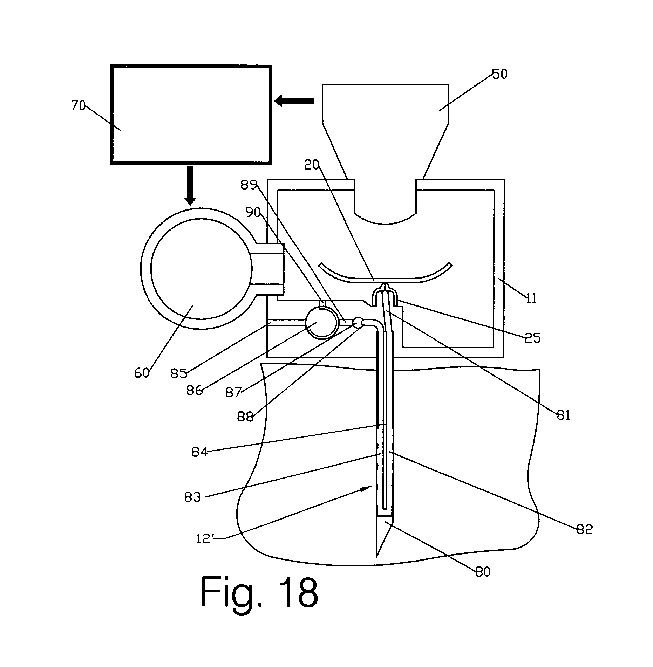

Fig. 18 shows a fifth embodiment of a fluid handling system that uses vacuum within the device housing to draw sample liquid on demand from the catheter and deposit it on the test zone. The dual channel catheter (12') provides the fluid transfer means between the subcutaneous tissue and the test element (20). It includes a first channel (82) and a second channel (83) separated by a barrier (84). The catheter may have a rigid, sharpened tip portion (80) for introduction if the catheter into tissue through the skin. Flow passage (81) connects channel (82) to the test strip. A rubber duckbill check valve (25) is attached to the end of flow passage (81) to allow fluid flow out to the test area, but prevent airflow in. It has the further characteristic of non-zero opening pressure. Flow passage (89) connects channel (83) to control valve (86). In a first position control valve (86) connects passage (89) to vent (90) within housing (11). In a second position it connects to vent (85) outside housing (11). Window (87) in flow passage (89) facilitates detection of liquid. Vacuum pump (60) reduces the pressure inside housing (11) that contains the test element. The control module (70)regulates the vacuum pump and the position of control valve. It may use optical information from optical module (50) and information on the presence of liquid at window (87) as input. In the condition shown in Fig. 18 passages (81), (82), (83) and a portion of (84) are filled by interstitial fluid. The fluid terminates at a fluid-air interface (88), and balance of flow passage (89), valve (86) and vents (85) and (90) are filled with air.

The test element and optical module (50) are the same as in the description of the first embodiment.

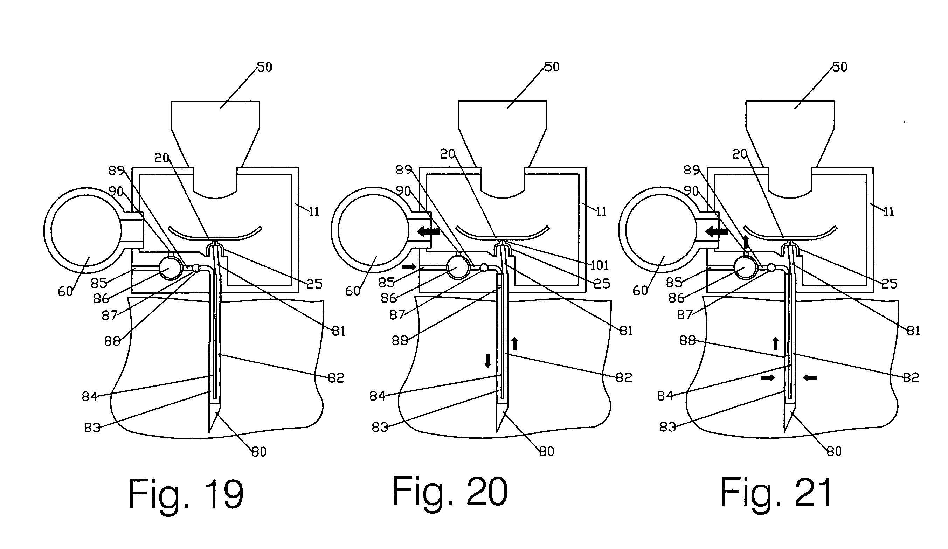

Operation is illustrated in Figures 19, 20 and 21. In Fig. 19 the catheter passages are filled with interstitial fluid and a fresh test zone is moved adjacent to the end of the duckbill valve. Fig. 20 shows delivery of a quantity of interstitial fluid in short time relative to the longer time required to collect this fluid from the tissue. Control valve (86) is moved to a second position so that passage (89) is connected to vent (85), and the vacuum pump is turned on. The difference between the reduced pressure in the interior of housing (11) and atmospheric pressure in vent (85) opens the duckbill valve and causes the interstitial fluid in channels (82) and (83) to flow out the duckbill valve opening. Here it forms a droplet (101) that contacts the test zone. In the process, air is drawn into channel (83) to replace the fluid volume that forms the droplet. When sufficient fluid has contacted the test zone, control valve (86) is shifted such that passage (89) is again connected to vent (90) as shown in Fig. 21. In this mode reduced pressure in the housing draws liquid into the catheter from the subcutaneous tissue, and displaces air through vent (90). This may be a slow process compared to the fast delivery of the sample to the test zone. The non-zero opening pressure of duckbill valve (25) limits the flow to the path exiting through vent (90). The vacuum is reduced when fluid is detected in window (87) to prevent further fluid withdrawal from the tissue.

Glucose measurement proceeds as in the first embodiment in Fig. 1.

A salient feature of embodiment 5 is that most of the fluid is stored within a porous catheter where it is in substantial equilibrium with the fluid in the surrounding tissue. This equilibrated sample is then quickly withdrawn on demand and measured, resulting in near real-time results. This is preferable to withdrawing the fluid from the tissue slowly and storing it in isolation from the tissue.

Although preferred embodiments of the invention have been described using specific terms, such description is for illustrative purposes only, and it is to be understood that changes and variations may be made without departing from the spirit or scope of the following claims.

## Claims

1. System for sampling body fluids, comprising
- an implantable catheter (12) for sampling body fluid
- a pump means for transporting a volume of said body fluid out of the catheter,
- a volume determination unit for determining said volume of fluid
- a control means (70) for controlling the pump means based on the determined volume of body fluid.

2. System according to claim 1, wherein the pump means comprises a vacuum means (60) for applying a suction to the catheter.

3. System according to claim 1 which comprises a measurement device for determination of an analyte concentration.

4. System according to claim 1, wherein the system employs a test element having a test zone for one way measurement.

5. System according to claim 1 or 4, wherein the test element provides multiple test zones.

6. System according to claim 5, wherein said test element is tape shaped or disc shaped.

7. System according to claim 5 or 6, wherein the system comprises a transport means for transporting fresh test areas into a contacting position for contacting a test area with fluid from the catheter.

8. System according to claim 1, wherein the volume determination unit determines the sampled volume, compares said volume with a desired value and controls the pump means such that subsequently sampled volumes are closer to the desired volume.

9. System according to claim 1 or 8, wherein said volume determination is made by optical determination of a test zone wetted with sample.

10. System according to claim 10, wherein said test element has a mesh layer with substantial rectangular or quadratic meshes.

11. System according to claim 10, wherein the opening of a single mesh is between 10 micrometer and 50 micrometer.

12. System according to claim 10, wherein said mesh is coated or impregnated with a material that renders the mesh hydrophilic, prefereably that material is an N-oleoyl-sarcosinate.

13. System according to claim 1, wherein the catheter samples body fluid without the need for non-bodily fluids as perfusion fluid.

14. System according to claim 1 , wherein the catheter is a single channel catheter.

15. System according to claim 1, wherein the catheter is a dual channel catheter having a channel that during withdrawal of fluid from the catheter is at least partially filled with air or gas.

16. System according to claim 1, wherein the pump means sequentially transports fluid boluses from the catheter.

17. System for sampling body fluids, comprising
- an implantable catheter (12) for withdrawing body fluid,
- a pump means for transporting a volume of body fluid out of the catheter,
wherein said pump means withdraws fluid with an overall flow rate of below than 10 nanoliters per minute from the catheter.

18. System for sampling body fluids, comprising
- an implantable catheter (12) for withdrawing body fluid,
- a pump means for transporting a volume of body fluid out of the catheter,
wherein said pump means in a single sampling event samples a fluid amount of below than 50 nanoliters, prefereably below than 15 nanoliters.

19. System according to claim 17 or 18, wherein said pump means comprises a shuttle (23) that moves within a bore to transport fluid.

20. System according to claim 19, wherein said shuttle is moved by magnetic force.

21. System according to claim 17 or 18, wherein said pump means comprises a plunger (33) moving in a bore and a vacuum pump acting on the plunger to move it in the bore.

22. System according to claim 21, wherein said pump means further comprises a spring (37) that moves the plunger backwards after it had been displaced by the vacuum pump.

23. System according to claim 17 or 18, wherein said pump means comprises a first pinch valve (44), second pinch valve 45 and pump clamps (46), said first pinch valve and said pump clamps are acting on a squeezable tube (43).

24. System for sampling body fluids, comprising
- an implantable dual channel catheter (12') for withdrawing body fluid,
- a pump means for transporting a volume of body fluid from a first channel (82) of the catheter and
- vent means for venting a second channel (83) of the catheter with air or gas.

25. System according to claim24, wherein said system further has a control valve (86) that in a first position connects the second channel to the pump means and in a second position connects the second channel to a vent for venting said second channel with air or gas.
